(19) Europäisches Patentamt
European Patent Office
Office européen des brevets

(11) Veröffentlichungsnummer: **0 176 806**
**A1**

(12) # EUROPÄISCHE PATENTANMELDUNG

(21) Anmeldenummer: **85111313.4**

(22) Anmeldetag: **06.09.85**

(51) Int. Cl.[4]: **C07D 513/04**, A61K 31/54, //(C07D513/04,285:00,249:00)

(30) Priorität: **08.09.84 DE 3433037**

(43) Veröffentlichungstag der Anmeldung: **09.04.86 Patentblatt 86/15**

(84) Benannte Vertragsstaaten: **AT CH DE FR GB IT LI**

(71) Anmelder: **GÖDECKE AKTIENGESELLSCHAFT**
**Salzufer 16**
**D-1000 Berlin 10(DE)**

(72) Erfinder: **Barth, Hubert, Dr.**
**Rosenweg 60**
**D-7830 Emmendingen(DE)**
Erfinder: **Hartenstein, Johannes, Dr.**
**Fohrenbühl 23**
**D-7801 Stegen-Wittental(DE)**
Erfinder: **Satzinger, Gerhard, Dr.**
**Im Mattenbühl 7**
**D-7809 Denzlingen(DE)**
Erfinder: **Fritschi, Edgar, Dr.**
**Am Scheuerwald 2**
**D-7811 St. Peter(DE)**
Erfinder: **Osswald, Hartmut, Prof. Dr.**
**Kiefernweg 1**
**D-7808 Waldkirch 2(DE)**
Erfinder: **Bartoszyk, Gerd**
**Buchenweg 6**
**D-7808 Waldkrich(DE)**

(54) **1,2,4-Benzothiadiazinoxid-Derivate, Verfahren zu deren Herstellung und diese enthaltende Arzneimittel.**

(57) Es werden 1,2,4-Benzothiadiazinoxid-Derivate der allgemeinen Formel I

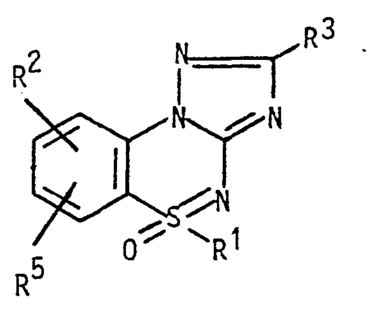

(I)

in welcher
$R^1$ einen Arylrest,
$R^2$ und $R^5$, die gleich oder verschieden sein können, ein Wasserstoff- oder Halogenatom oder eine Nitrogruppe und
$R^3$ ein Wasserstoffatom, eine Hydroxylgruppe, einen unsubstituierten oder durch Halogen, Amino-, Alkylamino-, Dialkylamino- oder Hydroxylgruppen substituierten, niederen Alkyl-, Alkoxyalkyl- oder Alkoxycarbonylrest mit 1 bis 4 Kohlenstoffatomen, oder einen unsubstituierten oder durch Halogenatome substituierten Phenylrest bedeuten.

beschrieben; ein weiterer Gegenstand sind Analogieverfahren zu deren Herstellung. Die gemäß der Verfahren hergestellten Verbindungen der allgemeinen Formel I besitzen wertvolle pharmakologische Eigenschaften, insbesondere zeichnen sie sich durch bemerkenswerte anxiolytische Wirkungen auf das Zentralnervensystem aus.



Rank Xerox

# 1,2,4-Benzothiadiazinoxid-Derivate Verfahren zu deren Herstellung und diese enthaltende Arzneimittel

Gegenstand der vorliegenden Erfindung sind 1,2,4-Benzothiadiazinoxid-Derivate der allgemeinen Formel I

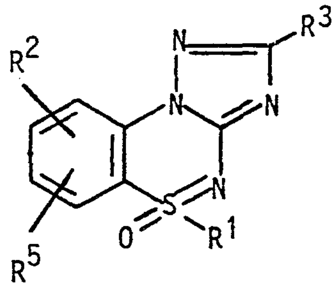

(I)

in welcher

$R^1$ einen unsubstituierten oder substituierten Arylrest,

$R^2$ und $R^5$, die gleich oder verschieden sein können, ein Wasserstoff- oder Halogenatom oder eine Nitrogruppe und

$R^3$ ein Wasserstoffatom, eine Hydroxylgruppe, einen unsubstituierten oder durch Halogen, Amino-, Alkylamino-, Dialkylamino- oder Hydroxylgruppen substituierten, niederen Alkyl-, Alkoxyalkyl oder Alkoxycarbonylrest mit 1 bis 4 Kohlenstoffatomen, oder einen unsubstituierten oder durch Halogenatome substituierten Phenylrest bedeuten.

Arylreste im Sinne der Erfindung sind gegebenenfalls substituierte Phenylreste. Bevorzugt ist der unsubstituierte sowie der gegebenenfalls durch Halogen oder durch eine niedere Alkoxygruppe monosubstituierte Phenylrest. Als Halogenatome kommen Fluor-, Chlor- und Bromatome infrage.

Bevorzugt sind 1,2,4-Benzothiadiazin-Derivate der allgemeinen Formel I, in welcher $R^1$ einen Phenyl-, Methoxyphenyl-, Fluorphenyl- oder Chlorphenylrest, $R^2$ und $R^5$, die gleich oder verschieden sein können, ein Wasserstoff-, Fluor-, Chlor-, Bromatom oder eine Nitrogruppe und $R^3$ ein Wasserstoffatom, eine Hydroxylgruppe oder einen Methyl-, Ethyl-, Ethoxyethyl-, Methoxymethyl-, Trifluormethyl-, Hydroxymethyl-, Ethoxycarbonyl-, Dimethylaminomethyl-, Phenyl-, Fluorphenyl-oder Chlorphenylrest bedeuten.

Die Substituenten $R^2$ und $R^5$ befinden sich bevorzugt in 7- oder 8-Stellung des Triazolo-1,2,4-benzothiadiazinsystems.

Ein weiterer Gegenstand der Erfindung ist ein Verfahren zur Herstellung von Verbindungen der allgemeinen Formel I, das dadurch gekennzeichnet ist, daß man in an sich bekannter Weise eine Verbindung der allgemeinen Formel II

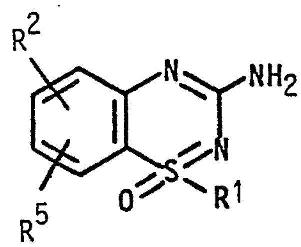

(II)

in welcher $R^1$, $R^2$ und $R^5$ die oben genannte Bedeutung haben,

mit einem Orthoester der allgemeinen Formel III

$R^3C(OR^4)_3$ (III)

in welcher $R^3$ die oben genannte Bedeutung hat und $R^4$ für einen niederen Alkylrest steht,

zu einer Verbindung der allgemeinen Formel IV

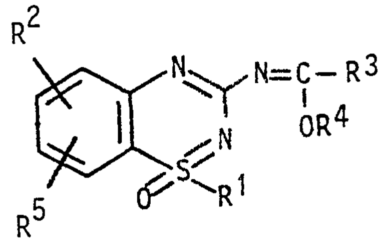

( IV )

in welcher $R^1$, $R^2$, $R^3$ und $R^5$ die oben genannte Bedeutung besitzen, und $R^4$ für einen niederen Alkylrest steht,

umsetzt, diese Verbindung ohne Isolierung mit Ammoniak in eine Verbindung der allgemeinen Formel V

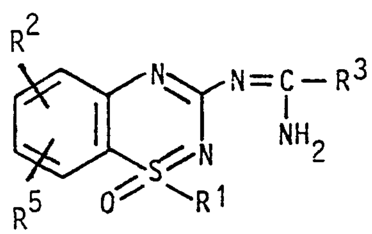

( V )

in welcher $R^1$, $R^2$, $R^3$ und $R^5$ die oben genannte Bedeutung haben,

überführt, und diese Verbindung in an sich bekannter Weise oxidativ cyclisiert.

Die als Ausgangsmaterial verwendeten Verbindungen der allgemeinen Formel II sind bekannt (Chem. Ber. 109, 2097, 1976) oder werden in Analogie zu den dort beschriebenen Verfahren hergestellt.

Das Verfahren zur Herstellung von Verbindungen der allgemeinen Formel I wird in Analogie zur DE-OS 2055889 bzw. DE-OS 2065714 durchgeführt.

Hierbei wird zunächst ein 3-Amino-1,2,4-benzothiadiazin-Derivat gemäß allgemeiner Formel II mit einem Orthoester der allgemeinen Formel III umgesetzt. Diese Umsetzung erfolgt zweckmäßigerweise ohne Lösungsmittel in einem bis zu 10fachen Überschuß an Orthoester bei einer Temperatur von 120° bis 160° C. Die Verwendung eines sauren Katalysators wie z.B. Essigsäure oder Salzsäure wirkt sich günstig auf die Umsetzung aus. Der Katalysator kann auch in Form eines Säureadditionssalzes der Ausgangsverbindung verwendet werden. Die Reaktionszeit beträgt 0,5 bis 2 Stunden. Nach der Reaktion wird der Orthoester im Wasserstrahlvakuum entfernt. Danach wird der 3-Imidoesterrest in einen 3-Amidinorest überführt. Diese Umwandlung geschieht zweckmäßigerweise mit einer Lösung von Ammoniak in einem niederen Alkohol bei Raumtemperatur. Vorzugsweise verwendet man Ethanol als Lösungsmittel. Die Reaktionszeit beträgt in der Regel 2 bis 20 Stunden. Während der Reaktion ausgefallenes Ausgangsmaterial wird abfiltriert, und das Filtrat nach Einengen und Lösen des Rückstandes in einem geeigneten Lösungsmittel der oxidativen Cyclisierung unterworfen. Als Lösungsmittel verwendet man bevorzugt Toluol, als Oxidationsmittel vorteilhaft Bleitetraacetat. Ein Zusatz von Essigsäure zur Reaktionsmischung wirkt sich vorteilhaft aus. Die Cyclisierungsreaktion wird zweckmäßigerweise bei Raumtemperatur durchgeführt. Die gemäß dem Verfahren erhaltenen 2-Alkyl-triazolo-1,2,4-benzothiadiazine werden durch Säulenchromatographie abgetrennt und/oder durch Kristallisieren gereinigt.

Eine weitere bevorzugte Variante des Herstellungsverfahrens ist dadurch gekennzeichnet, daß man wiederum ausgehend von einer Verbindung der allgemeinen Formel II diese in an sich bekannter Weise mit Mesitylensulfonylhydroxylamin (MSH) zu einer Verbindung der allgemeinen Formel VI

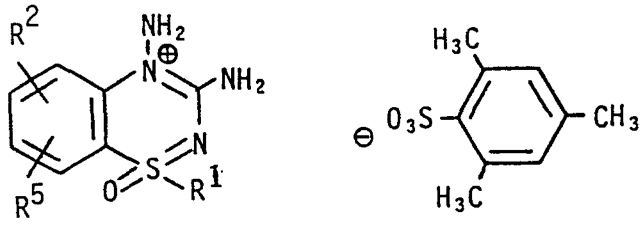

( VI )

in welcher $R^1$, $R^2$ und $R^5$ die oben genannte Bedeutung haben, umsetzt und das so erhaltene 1-Aryl-3,4-diamino-1,2,4-benzothiadiazinium-1-oxid Mesitylensulfonat (VI) entweder direkt oder nach Überführen in die Base VII

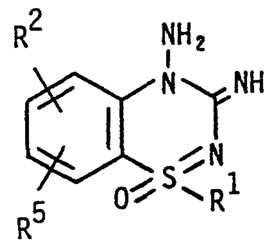

(VII)

in welcher $R^1$, $R^2$ und $R^5$ die oben genannte Bedeutung haben, gegebenenfalls in Gegenwart einer geeigneten Base, entweder mit einem geeigneten Carbonsäurederivat der allgemeinen Formel VIII

$R^3$-COX (VIII)

wobei $R^3$ die oben genannte Bedeutung hat und X für eine OH-Gruppe, ein Halogenatom oder eine Alkoxygruppe steht, oder mit einem Orthoester der allgemeinen Formel III zu einer Verbindung der allgemeinen Formel I umsetzt. Am Endprodukt kann erforderlichenfalls noch mittels eines geeigneten Metallhydrids der Rest $R^3$ reduziert werden.

Diese Verfahrensvariante wird in Analogie zu J. Het. Chem. 21, 1571, 1984 durchgeführt. Und zwar wird eine Verbindung der allgemeinen Formel II in einem organischen Lösungsmittel, bevorzugt Dichlormethan, bei 0° C mit Mesitylensulfonylhydroxylamin umgesetzt. Der ausgefallene und getrocknete Niederschlag der Verbindung der allgemeinen Formel VI wird, entweder in die Base VII überführt oder direkt weiterverarbeitet. Zur Gewinnung der Base VII wird das Mesitylensulfonat VI in einem organischen Lösungsmittel, bevorzugt Dioxan suspendiert und mit einer wässrigen Alkalilauge umgesetzt. Nach Abtrennung des Niederschlages wird die Base durch Einbringen des Filtrates in Eiswasser ausgefällt und aus einem organischen Lösungsmittel, bevorzugt Methanol umkristallisiert. Das Mesitylensulfonat VI oder die Base VII werden, gegebenenfalls suspendiert in einem polaren organischen Lösungsmittel, bevorzugt Pyridin oder Tetrahydrofuran, und erforderlichenfalls in Gegenwart einer starken Base, bevorzugt Natriumhydrid, mit einem berschuß einer Verbindung der allgemeinen Formeln III oder VIII unter Rückfluß zum Sieden erhitzt. Die verfahrensgemäß erhaltenen Verbindungen der allgemeinen Formel I werden dann nach Destillation des Lösungsmittels durch Verteilungschromatographie und/oder durch Kristallisation gereinigt. Erforderlichenfalls kann der Rest $R^3$ noch durch ein geeignetes Metallhydrid, bevorzugt Lithiumaluminiumhydrid, in einem inerten Lösungsmittel, bevorzugt Tetrahydrofuran, reduziert werden.

Die bei den Beispielen aufgeführten Verbindungen lassen sich nach beiden Verfahrensvarianten herstellen, doch führt die bevorzugte zweite Variante zu höheren Ausbeuten.

Die gemäß den Verfahrensvarianten hergestellten Verbindungen der allgemeinen Formel I besitzen wertvolle pharmakologische Eigenschaften, insbesondere zeichnen sie sich durch bemerkenswerte anxiolytische Wirkungen auf das Zentralnervensystem aus.

Ein weiterer Gegenstand der Erfindung sind daher Arzneimittel, enthaltend Verbindungen der allgemeinen Formel I, zur Bekämpfung von Angstzuständen.

Die erfindungsmäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B Tartrat- und Citrat-Puffer, Ethanol, Komplexbildner (wie Ethylendiamintetraessigsäure und deren nichttoxische Salze) sowie hochmolekulare Polymere (wie flüssiges Polyethylenoxid) zur Viskositätsregulierung. Feste Trägerstoffe sind z.B Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykol); für orale Applikation geeignete Zubereitungen können gewünschtenfalls zusätzliche Geschmacks-und/oder Süßstoffe enthalten.

Die enteral verabreichten Einzeldosen liegen im Bereich von etwa 5 bis 250 mg, vorzugsweise 10 - 100 mg. Parenteral werden etwa 1 bis 20 mg gegeben.

Die folgenden Beispiele sollen die Erfindung näher erläutern:

## Beispiel 1

(±)-7-Chlor-2-methyl-5-phenyl-[1,2,4]triazolo[5,1-c][1,2,4]benzothiadizin-5-oxid a.)

14,9 g (0,04 mol) 3-Amino-7-chlor-1-phenyl-1,2,4-benzothiadiazin-1-oxid Hydrobromid und 65 g Orthoessigsäuretriethylester werden 2 Stunden unter Rückfluß gekocht. Es bildet sich eine Suspension. Nach dem Abkühlen wird der Orthoessigsäureester im Wasserstrahlvakuum abdestilliert und der Rückstand mit ca. 15 %iger ethanolischer Ammoniaklösung (200 ml) über Nacht bei Raumtemperatur gerührt. Der Alkohol wird im Vakuum entfernt und der Rückstand mit 1 Liter Toluol und 20 ml Eisessig versetzt. Unter gutem Rühren gibt man 25,0 g Bleitetraacetat portionsweise hinzu und rührt dann 45 Minuten bei Raumtemperatur weiter. Man versetzt mit 500 ml Wasser, trennt die Toluolphase ab und wäscht sie mit wässriger Natriumhydrogencarbonatlösung. Nach dem Einengen wird der Rückstand an Kieselgel chromatographiert. Elutionsmittel Methylenchlorid / Methanol 20 : 1. Man erhält 5,1 g (±)-7-Chlor-2-methyl-5-phenyl-[1,2,4]triazolo[5,1-c][1,2,4]benzothiadiazin-5-oxid nach Kristallisation aus Isopropanol in Form farbloser Kristalle vom Fp. 207 °C.

In analoger Weise werden die folgenden Verbindungen erhalten:

(±)-2-Methyl-5-phenyl-[1,2,4]triazolo[5,1-c][1,2,4] benzothiadiazin-5-oxid b.) Fp. 212 °C, kristallisiert aus Toluol

(±)-7-Chlor-5-(3-chlorphenyl)-2-methyl-[1,2,4]triazolo[5-,1-c] [1,2,4]benzothiadiazin-5-oxid c.) Fp. 195-198 °C, kristallisiert aus Ethanol

(±)-7-Chlor-5-(4-chlorphenyl)-2-methyl-[1,2,4]triazolo[5-,1-c] [1,2,4]benzothiadiazin-5-oxid d.) Fp. 249-251 °C, kristallisiert aus Ethanol

Beispiel 2

(±)-8-Chlor-2-ethyl-5-phenyl[1,2,4]triazolo[5,1-c][1,2,4] benzothiadiazin-5-oxid a.)

2,9 g (9,9 mMol) 3-Amino-6-chlor-1-phenyl-1,2,4-benzothiadiazin-1-oxid, 15,0 g Orthopropionsäuretriethylester und 0,6 g Eisessig werden 1 Stunde unter Rückfluß erhitzt. Man kühlt ab, destilliert den überschüssigen Orthoester im Wasserstrahlvakuum ab und versetzt den Rückstand mit 50 ml einer ca. 15 %igen Lösung von Ammoniak in Ethanol. Man rührt 5 Stunden bei Raumtemperatur, filtriert den ausgefallenen Niederschlag ab und engt das Filtrat im Vakuum zur Trockene ein. Der Rückstand wird mit 100 ml Toluol und unter kräftigem Rühren bei Raumtemperatur portionsweise mit 4,0 g Bleitetraacetat versetzt.

Man rührt noch 20 Minuten bei Raumtemperatur, entfernt das Toluol am Rotationsverdampfer und verteilt den Rückstand zwischen Wasser und Chloroform. Die organische Phase wird abgetrennt, mit Wasser ausgewaschen und im Vakuum zur Trockene eingeengt. Der Rückstand wird zunächst an Kieselgel mit Chloroform/Methanol als Elutionsmittel, dann erneut an Kieselgel mit Toluol/Ethanol 10 : 1 als Elutionsmittel chromatographiert.

Die Hauptfraktion kristallisiert nach Anreiben mit Ether durch. Man erhält ein farbloses (±)-8-Chlor-2-ethyl-5-phenyl[1,2,4]triazolo[5,1-c][1,2,4]benzothiadiazin-5-oxid, Fp. 150 °C.

In analoger Weise erhält man:

(±)-8-Chlor-2-methyl-5-phenyl-[1,2,4]triazolo[5,1-c][1,-2,4] benzothiadiazin-5-oxid b.) Fp. 200 °C,

(±)-7-Chlor-2-methyl-5-(2-chlorphenyl)[1,2,4]triazolo[5,-1-c] [1,2,4]benzothiadiazin-5-oxid c.) Fp. 196 °C. Die Verbindung wurde aus Isopropanol kristallisiert und enthält 0,25 Mol Isopropanol.

(±)-7-Chlor-2-ethyl-5-phenyl-[1,2,4]triazolo[5,1-c][1,2,-4] benzothiadiazin-5-oxid d.) Fp. 153 °C, kristallisiert aus Isopropanol

Beispiel 3

(±)-7-Chlor-5-phenyl-2-trifluoromethyl-[1,2,4]triazolo[5,1-c] [1,2,4]-benzothiadiazin-5-oxid a.)

Eine Suspension von 18,2 g 3-Amino-7-chlor-1-phenyl-1,2,4-benzothiadiazin-1-oxid in 75 ml Dichlormethan wird unter Rühren bei 0 °C langsam mit einer Lösung von 16,1 g Mesitylensulfonylhydroxylamin (MSH) in 90 ml Dichlormethan versetzt. Nach beendeter Zugabe wird 1 Stunde bei Raumtemperatur weitergerührt und der ausgefallene Niederschlag abgesaugt. Nach Waschen mit Diethylether wird der Niederschlag an der Luft getrocknet. Man erhält farbloses 3,4-Diamino-7-Chlor-1-phenyl-1,2,4-benzothiadiazinium-1-oxid Mesitylensulfonat, Fp. 225 °C. Hiervon werden 10,0 g in 30 ml Pyridin suspendiert und unter Rühren mit 8,3 g Trifluoressigsäureanhydrid versetzt. Man kocht 4 Stunden, destilliert das Lösungsmittel vorsichtig im Wasserstrahlvakuum ab und verteilt den Rückstand zwischen Dichlormethan und wäßriger Natriumhydrogencarbonatlösung. Die organische Phase wird eingeengt und der Rückstand aus Ethanol kristallisiert. Es werden 3,9 g farbloses Produkt, Fp. 167 °C erhalten.

In analoger Weise erhält man die folgenden Verbindungen:

(±)-7-Brom-8-chlor-2-methyl-5-phenyl-[1,2,4]triazolo[5,-1-c] [1,2,4]-benzothiadiazin-5-oxid b.) Fp. 248 °C, kristallisiert aus Toluol

(±)-7-Fluor-2-methyl-5-phenyl-[1,2,4]triazolo[5,1-c][1,2-,4] benzothiadiazin-5-oxid c.) Fp. 205 °C kristallisiert aus Ethanol

(±-7-Chlor-5-(2-fluorphenyl)-2-methyl-[1,2,4]triazolo[5,-1-c] [1,2,4]benzothiadiazin-5-oxid d.) Fp. 230 °C aus Ethanol beige Kristalle

(±)-2-Methyl-7-nitro-5-phenyl-[1,2,4]triazolo[5,1-c][1,2,-4] benzothiadiazin-5-oxid e.) Fp. 255-258 °C aus Aceton gelbe Kristalle

(±)-7-Chlor-5-(2-methoxyphenyl)-2-methyl-[1,2,4]triazolo-[5,1-c][1,2,4]benzothiadiazin-5-oxid f.) Fp. 222-225 °C aus Essigester

Beispiel 4

(±)-7-Chlor-5-phenyl-[1,2,4]triazolo[5,1-c] [1,2,4]benzothiadiazin-5-oxid a.)

Eine Mischung von 7,2 g 3,4-Diamino-7-chlor-1-phenyl-1,2,4-benzothiadiazinium-1-oxid Mesitylensulfonat, 9,4 ml Pyridin und 12,5 ml Ameisensäure wird 5 Stunden unter Rückfluß erhitzt. Die dunkle Reaktionsmischung wird im Wasserstrahlvakuum eingeengt und der Rückstand zwischen Dichlormethan und Wasser verteilt. Die organische Phase wird abgetrennt, zur Trockene eingeengt, und der Rückstand aus Isopropanol/Diisopropylether kristallisiert. Man erhält 1,8 g farblose Kristalle, Fp. 193 °C.

In analoger Weise erhält man:

(±)-7-Brom-8-chlor-5-phenyl-[1,2,4]triazolo[5,1-c][1,2,-4] benzothiadiazin-5-oxid b.) Fp. 264 °C, kristallisiert aus Ethanol

Beispiel 5

(±)-7-Chlor-2,5-diphenyl-[1,2,4]triazolo[5,1-c] [1,2,4]benzothiadiazin-5-oxid a.)

10,1 g 3,4-Diamino-7-chlor-1-phenyl-1,2,4-benzothiadiazinium-1-oxid Mesitylensulfonat werden eine Stunde unter Rühren in 20 ml Benzoylchlorid rückflussiert. Das überschüssige Säurechlorid wird im Wasserstrahlvakuum abdestilliert, der Rückstand mit etwas Ethanol verrieben und der ausgefallene Niederschlag abgesaugt. Nach Kristallisieren aus Toluol erhält man 3,6 g farblose Kristalle, Fp. 271 °C.

In analoger Weise erhält man:

(±)-7-Chlor-2-(4-chlorphenyl)-5-phenyl-[1,2,4]triazolo[5-,1-c] [1,2,4]benzothiadiazin-5-oxid b.) Fp. 261-262 °C, kristallisiert aus Acetonitril

Beispiel 6

(±)-7-Chlor-2-ethoxocarbonyl-5-phenyl-[1,2,4]triazolo[5,1-c] [1,2,4]benzothiadiazin-5-oxid a.)

18,2 g 3-Amino-7-chlor-1-phenyl-1,2,4-benzothiadiazin-1-oxid werden unter Rühren bei 0 °C langsam mit einer Lösung von 16,1 g Mesitylensulfonylhydroxylamin in 90 ml Dichlormethan versetzt. Danach rührt man 1 Stunde bei Raumtemperatur weiter, saugt den ausgefallenen Niederschlag ab und wäscht gut mit Diethylether aus. Der trockene Niederschlag (30,4 g) wird in 200 ml Dioxan suspendiert und unter Rühren bei 0 °C mit 60 ml 20 %iger wäßriger Natronlauge versetzt. Man rührt 3 Stunden bei Raumtemperatur weiter, saugt den ausgefallenen Niederschlag ab und gießt das Filtrat in Eiswasser. Der ausgefallene Niederschlag wird abgesaugt, mit Wasser ausgewaschen und aus Ethanol kristallisiert. Man erhält 16,0 g farbloses 4-Amino-7-chlor-3,4-dihydro-3-imino-1-phenyl-1,2,4-benzothiadiazin-1-oxid, Fp. 168 °C. Davon werden unter Rühren 6,5 g in 25 ml Oxalsäureethylesterchlorid 5 Stunden auf 80 °C erhitzt. Das überschüssige Säurechlorid wird abdestilliert und der Rückstand zwichen Dichlormethan und wässriger Natriumhydrogencarbonatlösung verteilt. Das organische Lösungsmittel wird im Vakuum abdestilliert und der Rückstand aus Ethanol, dann aus Toluol und nochmals aus Ethanol kristallisiert. Man erhält 3,5 g farblose Kristalle mit einem Schmelzpunkt von 239 °C.

In analoger Weise erhält man:

(±)-7-Chlor-2-methoxymethyl-5-phenyl-[1,2,4]triazolo-[5,1-c] [1,2,4]benzothiadiazin-5-oxid b.) Fp. 222-225 °C aus Essigester

(±)-7-Chlor-2-hydroxymethyl-5-phenyl-[1,2,4]triazolo-[5,1-c] [1,2,4]benzothiadiazin-5-oxid c.) Fp. 222-225 °C aus Essigester, wird erhalten durch Reduktion von 6a.) mittels Lithiumaluminiumhydrid.

Beispiel 7

(±)-7-Chlor-2-dimethylaminomethyl-5-phenyl-[1,2,4]triazolo [5,1-c][1,2,4]benzothiadiazin-5-oxid

4,5 g 4-Amino-7-chlor-3,4-dihydro-3-imino-1-phenyl-1,2,4-benzothiadiazinium-1-oxid, hergestellt entsprechend Beispiel 6, und 3,9 g Dimethylaminoorthoessigsäuretrimethylester werden mit 3 ml Essigsäure versetzt und 5 Stunden auf 80 °C erhitzt. Die flüchtigen Bestandteile werden im Wasserstrahlvakuum abdestilliert und der Rückstand mit Essigester verrieben. Das ausgefallene Produkt wird abgesaugt und zweimal an Kieselgel mit Dichlormethan/Methanol 100 : 5 als Elutionsmittel chromatographiert. Die Hauptfraktion wird eingeengt und aus Essigester kristallisiert. Man erhält 2,3 g farblose Kristalle mit einem Schmelzpunkt von 202 °C.

Beispiel 8

(±)-7-Chlor-2-hydroxy-5-phenyl-[1,2,4]triazolo [5,1-c][1,2,4] benzothiadiazin-5-oxid

7,0 g 4-Amino-7-chlor-3,4-dihydro-3-imino-1-phenyl-1,2,4-benzothiadiazinium-1-oxid, hergestellt analog Beispiel 6, werden in 250 ml Tetrahydrofuran gelöst und mit 0,68 g Natriumhydrid (80 %ig) portionsweise versetzt. Man rührt 30 Minuten bei Raumtemperatur und tropft dann 2,4 ml Chlorameisensäureethylester, gelöst in 10 ml Tetrahydrofuran hinzu. Man rührt 3 Stunden bei Raumtemperatur, filtriert das ausgefallene Natriumchlorid ab, engt das Filtrat ein und nimmt den Rückstand in 150 ml Toluol auf. Man kocht 16 Stunden unter Rückfluß, saugt das ausgefallene Reaktionsprodukt ab und kristallisiert aus Dimethylformamid / Methanol farblose Kristalle, Fp. 343-348 °C.

Zur Erläuterung der pharmakologischen Wirksamkeit der Verbindungen gemäß allgemeiner Formel I wurden folgende Vergleichsversuche durchgeführt:

Vergleichsversuche

Methoden:

1. Versuchstiere

Versuchstiere waren männliche Mäuse (NMRI, Herkunft: Ivanovas, Kissleg) mit einem Gewicht von 18 bis 25 g. Die Tiere wurden bei Futter und Wasser ad lib. gehalten und je nach Versuch 2 bis 16 Stunden vor Versuchsbeginn bei Wasser ad lib. nüchtern gesetzt.

2. Substanzen

Die zu prüfenden Substanzen wurden in einer 0,8 prozentigen Methocelsuspension 30 Minuten vor dem Versuchsbeginn oral mittels Schlundsonde gegeben.

3. Versuchsdurchführung

3.1 Anxiolytische Wirkung

Die Modelle zur Prüfung auf anxiolytische Wirkung gehen davon aus, daß beim Tier experimentell ein Konflikt dadurch erzeugt wird, daß dem natürlichen Explorationstrieb der Tiere ein angstinduzierender Stimulus (aversiver Fußschock, bzw. aversive Beleuchtung) entgegen gesetzt wird.

a) bestraftes Explorationsverhalten (4-Platten-Test):

Die Testbox besitzt einen Metallboden aus vier gleichgroßen Rechtecken. Nach dem Einsetzen des Tieres kann das Tier 15 Sekunden frei explorieren. Anschließend wird jeder Übergang von einer der Metallplatten des Bodens auf eine andere mit einem milden Fußschock (1,5 mA für 0,5 s) bestraft. Die Anzahl der innerhalb einer Minute tolerierten Fußschocks ist das Maß für die anxiolytische Wirkung einer Substanz. Die durchschnittliche Zahl der akzeptierten Fußschocks der Kontrolltiere wurde als 100 % gesetzt. Pro Dosierung wurden 10 Tiere untersucht.

b) unbestraftes Explorationsverhalten (Hell-Dunkel-Präferenz):

Die Testanlage besteht aus einem größeren hell beleuchteten Abteil und einem kleineren dunklen Abteil. Das Tier wird in das dunkle Abteil gesetzt und für 10 Minuten beobachtet. Die Anzahl der Abteilwechsel und die Aufenthaltsdauer in den zwei Abteilen wird gemessen. Die durchschnittliche Anzahl der Abteilwechsel der Kontrolltiere wird als 100 % gesetzt. Pro Dosierung wurden 10 Tiere untersucht.

3.2 Motilitätstest

Die Spontanmotilität wurde gleichzeitig für eine Substanz- und eine Kontrollgruppe von je 5 Tieren in zwei undurchsichtigen Käfigen (Länge 50 cm, Breite 25 cm, Höhe 30 cm) mit Hilfe von jeweils drei Infrarot-Lichtschranken 30 Minuten lang gemessen. Die Summe der während dieser Zeit registrierten Impulse der Kontrollgruppe wurde gleich 100 gesetzt und die Abweichung bei den Substanzgruppen in % davon berechnet.

3.3 Narkosedauer-Versuch

70 mg/kg (10 ml/kg Körpergewicht) Hexobarbital-Natrium wurden innerhalb von 15 Sekunden in eine Schwanzvene injiziert. Pro Dosis wurden 10 Tiere verwendet. Als Kriterium für das Ende der Narkose wurde das Aufrichten aus der Seitenlage gewertet. Die durchschnittliche Narkosedauer der Kontrolltiere wurde gleich 100 gesetzt und die gemessenen Verlängerungen in % davon berechnet.

Die Ergebnisse der Versuche sind nachfolgend in der Tabelle I wiedergegeben.

TABELLE I

# TABELLE I

| Pharmakologische und biochemische Wirkungen | | | | | | |
|---|---|---|---|---|---|---|
| | Beispiel | | | | | |
| Modell | 1a | 1c | 2c | 4a | 4b | Diazepam |
| Anxiolyse (bestraftes Explorieren) | 7,5-45 mg/kg 30% bis 172% Zunahme | 15 mg/kg 35% Zunahme | 30 mg/kg 66% Zunahme | 15 mg/kg 45% Zunahme | 12,5 mg/kg 34% Zunahme | 1 - 4 mg/kg 32% bis 179% Zunahme |
| Anxiolyse (unbestraftes Explorieren) | 20-30 mg/kg 24% bis 40% Zunahme | | | | | 2 mg/kg 43% Zunahme 5 mg/kg 33% Abnahme |
| Spontan-motilität | 50 mg/kg 45% Zunahme | 15 mg/kg 12% Zunahme | 30 mg/kg 6% Zunahme | 100 mg/kg kein Effekt | 12,5 mg/kg 83% Zunahme | 5 mg/kg 67% Abnahme |
| Ataxie-Schwelle | > 100 mg/kg | > 100 mg/kg | > 100 mg/kg | > 100 mg/kg | > 400 mg/kg | 5mg/kg |
| Hypnotische Wirkung (Schlaf-zeitverlängerung) | 25 mg/kg 123% Zunahme 50 mg/kg 175% Zunahme | 15 mg/kg 6% Zunahme | 30 mg/kg 96% Zunahme | 100 mg/kg 224% Zunahme | 25 mg/kg 21% Zunahme | 2 mg/kg 286% Zunahme 4 mg/kg 320% Zunahme |
| Rezeptorbindung Benzodiazepin-Rez. GABA-Ratio | Kd = 1,5 µM R = 1,6 part. Ago. | Kd = 10 µM | Kd = 20 nM R = 1,1 part. Ago. | Kd = 3,5 µM R = 1,4 part. Ago. | Kd = 10 µM | Kd = 64 nM R = 2,9 Agonist |
| $LD_{50}$ | 1600 mg/kg | >400 mg/kg | > 400 mg/kg | > 400 mg/kg | > 400 mg/kg | 700 mg/kg |

" > ": kein Effekt bis zu dieser höchsten untersuchten Dosis

Die Verbindungen der allgemeinen Formel I zeigen anxiolytische Wirkungen im bestraften Explorationskonflikt (4-Platten-Test, Aron et al., Neuropharmacology 10, 459 - 469, 1970) und im unbestraften Explorationskonflikt (Hell-Dunkel-Präferenztest). So zeigt z. B. Beispiel 1a mit 7,5 bis 45 mg/kg eine anxiolytische Wirkung, welche mit der von Diazepam (1 - 4 mg/kg) vergleichbar ist. Während jedoch Diazepam bei 5 mg/kg bereits die Spontanmotilität um 67 % senkt, bewirken 50 mg/kg Beispiel 1a eine Steigerung der Motilität um 45 %. Auch die Schwelle für Ataxie liegt für Diazepam bei ca. 5 mg/kg, während die Verbindung Beispiel 1a bis 100 mg/kg nicht ataktisch wirkt.

**Ansprüche**

1) 1,2,4-Benzothiadiazinoxid-Derivate der allgemeinen Formel I

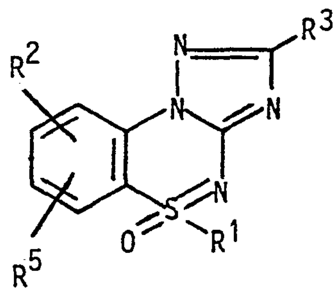

( I )

in welcher

$R^1$ einen unsubstituierten oder substituierten Arylrest,

$R^2$ und $R^5$, die gleich oder verschieden sein können, ein Wasserstoff- oder Halogenatom oder eine Nitrogruppe und

$R^3$ ein Wasserstoffatom, eine Hydroxylgruppe, einen unsubstituierten oder durch Halogen, Amino-, Alkylamino-, Dialkylamino- oder Hydroxylgruppen substituierten, niederen Alkyl-, Alkoxyalkyl-oder Alkoxycarbonylrest mit 1 bis 4 Kohlenstoffatomen, oder einen unsubstituierten oder durch Halogenatome substituierten Phenylrest bedeuten.

2.) 1,2,4-Benzothiadiazinoxid-Derivate gemäß Patentanspruch 1, dadurch gekennzeichnet daß

$R^1$ einen Phenyl-, Methoxyphenyl-, Fluorphenyl- oder Chlorphenylrest,

$R^2$ und $R^5$ ein Wasserstoff-, Chlor- Fluor- oder Bromatom oder eine Nitrogruppe und

$R^3$ ein Wasserstoffatom, eine Hydroxylgruppe oder einen Methyl-, Ethyl-, Methoxymethyl-, Trifluormethyl-, Ethoxyethyl-, Hydroxymethyl-, Ethoxycarbonyl-, Dimethylaminomethyl-, Phenyl-, Fluorphenyl- oder Chlorphenylrest bedeuten.

3.) 1,2,4-Benzothiadiazinoxid-Derivate gemäß einem der Ansprüche 1 oder 2, worin sich die Substituenten $R^2$ und $R^5$ in 7- oder 8-Stellung des Triazolo-1,2,4-Benzothiadiazinsystems befinden.

4.) (±)-7-Chlor-2-methyl-5-phenyl-[1,2,4]triazolo[5,1-c][1,2,4]benzothiadizin-5-oxid.

5.) (±)-7-Chlor-5-(3-chlorphenyl)-2-methyl-[1,2,4]triazolo[5,1-c][1,2,4]benzothiadiazin-5-oxid.

6.) (±)-7-Chlor-2-methyl-5-(2-chlorphenyl)[1,2,4]triazolo[5,1-c][1,2,4]benzothiadiazin-5-oxid.

7.) (±)-7-Chlor-5-phenyl-[1,2,4]triazolo[5,1-c][1,2,4]benzothiadiazin-5-oxid.

8.) (±)-7-Brom-8-Chlor-5-phenyl-[1,2,4]triazolo[5,1-c][1,2,4]benzothiadiazin-5-oxid.

9.) Verfahren zur Herstellung von Verbindungen der allgemeinen Formel I, dadurch gekennzeichnet, daß man in an sich bekannter Weise eine Verbindung der allgemeinen Formel II

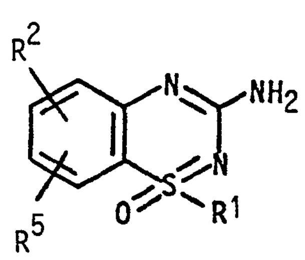

( II )

in welcher $R^1$, $R^2$ und R5 die oben genannte Bedeutung haben, entweder

a.) mit einem Orthoester der allgemeinen Formel III

$R^3C(OR^4)_3$ (III)

in welcher $R^3$ die oben genannte Bedeutung hat und $R^4$ für einen niederen Alkylrest steht, zu einer Verbindung der allgemeinen Formel IV

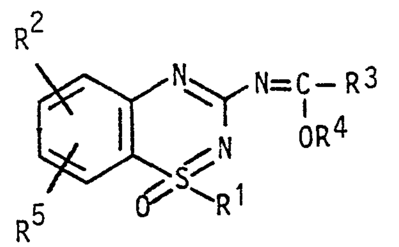

(IV)

in welcher $R^1$, $R^2$, $R^3$, $R^4$ und $R^5$ die oben genannte Bedeutung besitzen, umsetzt, diese Verbindung ohne Isolierung mit Ammoniak in eine Verbindung der allgemeinen Formel V

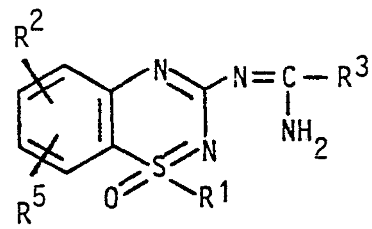

(V)

in welcher $R^1$, $R^2$, $R^3$ und $R^5$ die oben genannte Bedeutung haben, überführt, und diese Verbindung in an sich bekannter Weise oxidativ cyclisiert, oder

b.) mit Mesitylensulfonylhydroxylamin (MSH) zu einer Verbindung der allgemeinen Formel VI

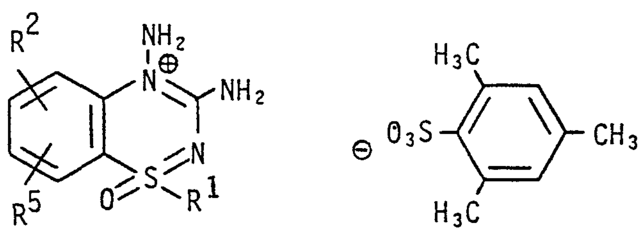

(VI)

umsetzt, in welcher $R^1$, $R^2$ und $R^5$ die oben genannte Bedeutung haben und das so erhaltene 1-Aryl-3, 4-diamono-1, 2,-benzothiadiazinium-1-oxid Mesitylensulfonat (IV) entweder direkt oder nach Überführen in die Base VII

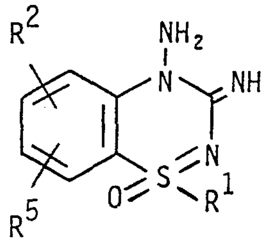

( VII)

in welcher $R^1$, $R^2$ und $R^5$ die oben genannte Bedeutung haben, und gegebenenfalls in Gegenwart einer geeigneten Base entweder mit einem geeigneten Carbonsäurederivat der allgemeinen Formel VIII

$R^3$-COX (VIII)

wobei $R^3$ die oben genannte Bedeutung hat und X für eine OH-Gruppe, ein Halogenatom oder eine Alkoxygruppe steht, oder mit einem Orthoester der allgemeinen Formel III zu einer Verbindung der allgemeinen Formel I umsetzt, in welcher man gegebenenfalls $R^3$ durch ein Metallhydrid reduziert.

10.) Arzneimittel enthaltend ein 1,2,4-Benzothiadiazinoxid-Derivat nach Anspruch 1 bis 8 nebst üblichen Hilfs- und Trägerstoffen.

Patentansprüche für den Vertragsstaat: AT

1.) Verfahren zur Herstellung von 1,2,4-Benzothiadiazinoxid-Derivaten der allgemeinen Formel I

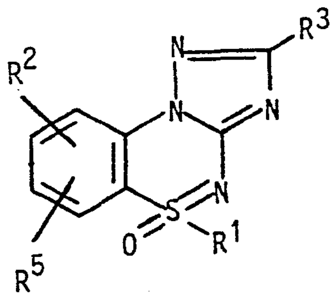

( I)

in welcher

$R^1$ einen unsubstituierten oder substituierten Arylrest,

$R^2$ und $R^5$, die gleich oder verschieden sein können, ein Wasserstoff- oder Halogenatom oder eine Nitrogruppe und

$R^3$ ein Wasserstoffatom, eine Hydroxylgruppe, einen unsubstituierten oder durch Halogen, Amino-, Alkylamino-, Dialkylamino- oder Hydroxylgruppen substituierten. niederen Alkyl-, Alkoxyalkyl-oder Alkoxycarbonylrest mit 1 bis 4 Kohlenstoffatomen, oder einen unsubstituierten oder durch Halogenatome substituierten Phenylrest bedeuten, dadurch gekennzeichnet, daß man in an sich bekannter Weise eine Verbindung der allgemeinen Formel II

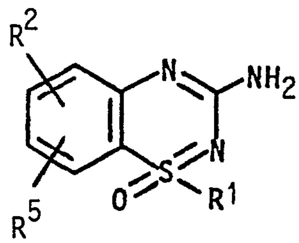

( II)

in welcher $R^1$, $R^2$ und $R^5$ die oben genannte Bedeutung haben, entweder

a.) mit einem Orthoester der allgemeinen Formel III

$R^3C(OR^4)_3$ (III)

in welcher $R^3$ die oben genannte Bedeutung hat und $R^4$ für einen niederen Alkylrest steht, zu einer Verbindung der allgemeinen Formel IV

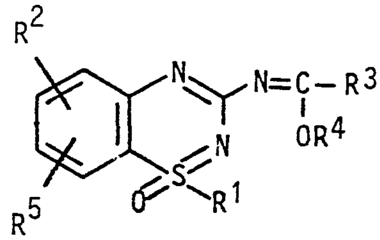

(IV)

in welcher $R^1$, $R^2$, $R^3$, $R^4$ und $R^5$ die oben genannte Bedeutung besitzen, umsetzt, diese Verbindung ohne Isolierung mit Ammoniak in eine Verbindung der allgemeinen Formel V

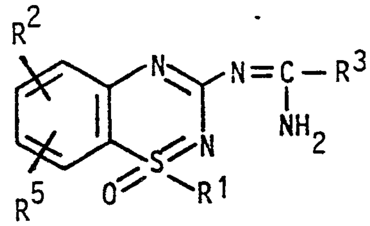

(V)

in welcher $R^1$, $R^2$, $R^3$ und $R^5$ die oben genannte Bedeutung haben, überführt, und diese Verbindung in an sich bekannter Weise oxidativ cyclisiert, oder

b.) mit Mesitylensulfonylhydroxylamin (MSH) zu einer Verbindung der allgemeinen Formel VI

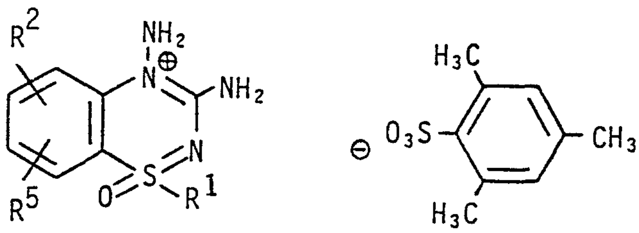

(VI)

umsetzt, in welcher $R^1$, $R^2$ und $R^5$ die oben genannte Beduutung haben und das so erhaltene 1-Aryl-3, 4-diamino- 1.2,4-benzothiadiazinium-1-oxid Mesitylensulfonat (VI) entweder direkt oder nach Überführen in die Base VII

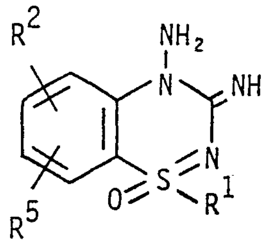

(VII)

in welcher $R^1$, $R^2$ und $R^5$ die oben genannte Bedeutung haben, und gegebenenfalls in Gegenwart einer geeigneten Base entweder mit einem geeigneten Carbonsäurederivat der allgemeinen Formel VIII

$R^3$-COX (VIII)

wobei $R^3$ die oben genannte Bedeutung hat und X für eine OH-Gruppe, ein Halogenatom oder eine Alkoxygruppe steht, oder aber mit einem Orthoester der allgemeinen Formel III zu einer Verbindung der allgemeinen Formel I umsetzt, und gegebenenfalls anschließend den Rest $R^3$ reduziert.

2.) Verfahren zur Herstellung von 1,2,4-Benzothiadiazinoxid-Derivate gemäß Patentanspruch 1, dadurch gekennzeichnet daß

$R^1$ einen Phenyl-, Methoxyphenyl-, Fluorphenyl- oder Chlorphenylrest,

$R^2$ und $R^5$ ein Wasserstoff-, Chlor- Fluor- oder Bromatom oder eine Nitrogruppe und

$R^3$ ein Wasserstoffatom, eine Hydroxylgruppe oder einen Methyl-, Ethyl-, Methoxymethyl-, Trifluormethyl-, Ethoxyethyl-, Hydroxymethyl-, Ethoxycarbonyl-, Dimethylaminomethyl-, Phenyl-, Fluorphenyl- oder Chlorphenylrest bedeuten.

3.) Verfahren zur Herstellung von 1,2,4-Benzothiadiazinoxid-Derivaten gemäß einem der Ansprüche 1 oder 2, worin sich die Substituenten $R^2$ und $R^5$ in 7- oder 8-Stellung des Triazolo-1,2,4-Benzothiadiazinsystems befinden.

4.) Verfahren zur Herstellung von (±)-7-Chlor-2-methyl-5-phenyl-[1,2,4]triazolo[5,1-c][1,2,4]benzothiadizin-5-oxid.

5.) Verfahren zur Herstellung von (±)-7-Chlor-5-(3-chlorphenyl)-2-methyl-[1,2,4]triazolo[5,1-c]-[1,2,4] benzothiadiazin-5-oxid.

6.) Verfahren zur Herstellung von (±)-7-Chlor-2-methyl-5-(2-chlorphenyl)[1,2,4]triazolo[5,1-c][-1,2,4] benzothiadiazin-5-oxid.

7.) Verfahren zur Herstellung von (±)-7-Chlor-5-phenyl-[1,2,4]triazolo[5,1-c][1,2,4]benzothiadiazin-5-oxid.

8.) Verfahren zur Herstellung von (±)-7-Brom-8-Chlor-5-phenyl-[1,2,4]triazolo[5,1-c][1,2,4]benzothiadiazin-5-oxid.

Europäisches Patentamt

**EUROPÄISCHER RECHERCHENBERICHT**

Nummer der Anmeldung

EP 85 11 1313

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | CHEMISCHE BERICHTE, Band 109, 1976, Seiten 2097-2106, Springer-Verlag, Heidelberg, DE; P. STOSS et al.: "3-Amino-1lambda4,2,4-benzothiadiazin-1-oxide" * Seite 2099, Zeilen 16-17, Formel 96 * --- | 1,10 | C 07 D 513/04<br>A 61 K 31/54 //<br>(C 07 D 513/04<br>C 07 D 285:00<br>C 07 D 249:00 ) |
| Y | DE-A-2 530 792 (GÖDECKE AG) * Seite 17; Seite 8, Zeilen 21-22 * ----- | 1,10 | |
| | | | **RECHERCHIERTE SACHGEBIETE (Int. Cl.4)** |
| | | | C 07 D 513/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 20-12-1985 | BACON, A.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82